# EUROPEAN PATENT APPLICATION

(11) **EP 2 870 941 A1**
(43) Date of publication of application: **13.05.2015**
(21) Application number: 13306532.6
(22) Date of filing: 08.11.2013
(51) Int. Cl.: A61B 19/00

(54) **Method for planning a surgical intervention**

(71) Applicant: Orthotaxy, 38700 La Tronche (FR)
(72) Inventor: Lavallee, Stéphane, 38410 St Martin D'Uriage (FR); Mersch, Guillaume, 38400 St Martin D'Heres (FR)
(74) Representative: Regimbeau

(57) **Abstract**

The invention relates to a method for planning a surgical intervention comprising the implantation of an implant in a patient's anatomical structure, comprising:
- computing at least one pseudo-radiographic image from a 3D image of the anatomical structure, said pseudo-radiographic image being a 2D image wherein each pixel integrates the information of the 3D image along a determined direction of integration, said determined direction of integration depending on the planned position of the implant with respect to the anatomical structure;
- displaying said at least one pseudo-radiographic image on a display unit;
- displaying a representation of the implant on said pseudo-radiographic image;
- updating the pseudo-radiographic image and/or the representation of the implant when the position of the implant is modified.

## Description

### FIELD OF THE INVENTION

The invention relates to a method for planning a surgical intervention comprising the implantation of an implant in a patient's anatomical structure.

### BACKGROUND OF THE INVENTION

The planning of a surgical intervention intended to place an implant in a patient's bone is currently done by the surgeon on the basis of a 3D bone model that allows the surgeon to visualize the morphology of the bone and possibly the implant positioned into the bone.

In order to provide such a 3D bone model to the surgeon, the current procedure generally consists in acquiring a 3D medical image (e.g. obtained by CT or MRI) of the patient, in sending said 3D medical image to an expert center wherein a precise segmentation of said image is carried out in order to generate the 3D bone model, and sending said model to the surgeon.

The expert center usually comprises experts (engineers and/or technicians) in the processing of medical images.

The experts use specific tools for facilitating the segmentation of the images. However, since the 3D medical image usually comprises a plurality of slices - typically from 150 to 200 slices - an error in the segmentation of a slice may generate a large error in the final result.

Hence, the segmentation cannot be completely carried out automatically, and the expert has to segment manually at least the regions of the 3D medical image where the greyscale impedes an automatic recognition of the pixels between bone and soft tissues.

Such a manual segmentation may take several hours and thus contributes to a high cost of the 3D model.

Besides, this process thus requires several flows of data, which is time-consuming and unpractical.

In addition, the 3D bone model that is obtained by the segmentation is not a medical image, which requires the surgeon to carry out the planning on an image that is not familiar to him.

### BRIEF DESCRIPTION OF THE INVENTION

A goal of the invention is thus to define a method for planning a surgical intervention that does not require any processing of the images by an expert center and that allows the surgeon to work on a type of images that is familiar to him or her and to get a more straightforward understanding of the information provided to him or her.

The invention provides a method for planning a surgical intervention comprising the implantation of an implant in a patient's anatomical structure, comprising:
- computing at least one pseudo-radiographic image from a 3D image of the anatomical structure, said pseudo-radiographic image being a 2D image wherein each pixel integrates the information of the 3D image along a determined direction of integration, said determined direction of integration depending on the planned position of the implant with respect to the anatomical structure;
- displaying said at least one pseudo-radiographic image on a display unit;
- displaying a representation of the implant on said pseudo-radiographic image;
- updating the pseudo-radiographic image and/or the representation of the implant when the position of the implant is modified.

By "anatomical structure" is meant in the present text a substantially rigid structure, such as a bone, whose shape can be determined on medical images and whose shape will not substantially evolve between the acquisition of the medical images and the planning of the surgical intervention. It can be but is not limited to an osseous structure.

The method thus allows the user to benefit from images that are familiar to him, since the pseudo-radiographic images and the representation of the implant that are displayed are similar to radiographic images onto which the surgeon visualizes the implant one implanted. Hence, the understanding of the displayed image by the surgeon is more straightforward.

In addition, the update can be done in real time when the position of the implant is modified.

According to an embodiment, the 3D image is a 3D medical image directly obtained by Computed Tomography.

According to an alternative embodiment, the 3D image is a 3D augmented medical image obtained by applying to a 3D medical image at least one of the following transformations:
- modifying the grey level values of the 3D medical image using a look-up table,
- creating a 3D model of the anatomical structure by an automatic segmentation of the 3D medical image, and assigning a grey level value to each voxel of said 3D model,
- creating a 3D model of the anatomical structure by an automatic segmentation of the 3D medical image, and assigning a grey level value to each voxel of said 3D model using a priori models of the anatomical structure, said a priori models comprising cortical bone models and spongious bone models,
- creating a 3D model of the anatomical structure by an automatic segmentation of the 3D medical image, and assigning grey level values to the external surface of said 3D model.

For creating said 3D augmented medical image, the 3D medical image may be a magnetic resonance image.

According to a preferred embodiment, said determined direction of integration is a specific direction of the implant, such as one of the three axes of the implant referential.

According to an embodiment, said determined direction of integration is defined by a specific direction of the implant and by at least one anatomical parameter such as a mechanical axis of a bone on which the implant shall be implanted.

The method may comprise computing at least two pseudo-radiographic images according to different directions of integration and displaying on the same display unit said at least two pseudo-radiographic images and a representation of the implant on each of said images.

The method may further comprise computing at least one slice of a 3D image and displaying a representation of the implant on said slice.

According to an advantageous embodiment, said slice is computed according to the same direction as the determined direction of integration of the pseudo-radiographic image and the method further comprises using a window for alternatively displaying the pseudo-radio image and said slice of the 3D image on the display unit.

The method may further comprise computing volume rendering of the 3D image and displaying said computed image with a representation of the implant on the same display unit as the at least one pseudo-radiographic image.

According to an advantageous embodiment, a reference feature of the implant may be highligthed on the volume rendering computed image.

The method may further comprise displaying selected anatomical landmarks on the pseudo-radiographic image.

Advantageously, the method comprises providing control elements for interactively modifying the position of the implant.

Said control elements may be displayed on the at least one pseudo-radiographic image.

According to a specific application of the method, the implant is a femoral or a tibial component of a knee prosthesis.

Another aspect of the invention is a computer program product comprising computer-readable instructions which, when loaded and executed on a suitable system, perform the steps of the method described above.

### BRIEF DESCRIPTION OF THE DRAWINGS

Other features and advantages of the invention will be apparent from the appended drawings, wherein:
- Figure 1 is a schematic view of an implant according to different directions of view;
- Figure 2 is a schematic view showing an example of a display comprising two pseudo-radiographic images with implant and anatomical structures (a, b), a slice with implant (c), a 3D volume rendered image with some pixels highlighted (d);
- Figure 3 is a zoom of figure 2 on a coronal pseudo-radiograph of the knee wherein the implant is displayed in transparent color.
- Figure 4 shows a coronal pseudo-radiograph of the knee wherein the implant is displayed in opaque white, as it would appear on post-surgical radiographies.
- Figure 5 shows a reconstructed image on which an anatomical reference such as knee center can be selected;
- Figure 6 shows the frontal (left) and sagittal (right) representation of a knee implant along the knee implant's axis.
- Figure 7 shows the frontal and sagittal representation of a knee implant along the 3D medical image axis if the implant is rotated with respect to the 3D medical image axis.
- Figure 8 is a zoom of figure 2 on a sagittal pseudo-radiograph.
- Figure 9 is a sagittal pseudo-radiograph with the same definitions of image axis as in figure 8, with the same input parameters including flessum, wherein some varus has been added.
- Figure 10 is an axial slice with the representation of the knee implant.
- Figure 11 is an axial slice with the representation of the knee implant with the same parameters for the position of the knee prosthesis as in figure 10, wherein some external rotation has been added.
- Figure 12 is a volume rendering of the 3D image with a representation of the implant, wherein the opaque pixels on the plane defined by prosthesis K, Y implant and Z implant have been colored.
- Figure 13 is a sagittal slice with the representation of the femur component of a knee implant with buttons to switch from a slice image to a pseudo-radiographic image back to a slice image.
- Figure 14 is a sagittal pseudo-radiographic image with the representation of the femur component of a knee implant with buttons to switch from a slice image to a pseudo-radiographic image back to a slice image. The image has been modified but the implant representation is the same.
- Figure 15 is an axial cut of the femur bone showing that there are two bumps on the anterior cortical, which makes it hard to identify the distance from the implant to the anterior cortical on a radio.
- Figure 16 is a pseudo-radiographic image with the representation of an implant wherein controls are displayed on the pseudo-radiographic image.
- Figure 17 shows a slice of the 3D image with the representation of an implant wherein clicking and dragging on the implant (the mouse move being represented by the arrow between the 2 black points) translates the implant accordingly.
- Figure 18 is a schematic view showing a native MR image of a bone joint.
- Figure 19 is the image of figure 18 wherein the bones have been segmented and all pixels of bone have been replaced by a value (white).
- Figure 20 is the image of figure 18 wherein the bones have been segmented and the lining has been replaced by a value (the same white as cortical bone) and the inners have been replaced by a different value (the same as spongious bone).
- Figure 21 is the image of figure 18 wherein the bones have been segmented and the lining has been replaced by a value (white) and the inners have not been replaced.
- Figure 22 shows a coronal pseudo-radiograph of the knee wherein the lining of the implant is displayed.
- Figure 23 is a schematic view of an implant (here, a tibial component of a knee prosthesis) according to different directions of view.

### DETAILED DESCRIPTION OF EMBODIMENTS OF THE INVENTION

The 3D medical image of the anatomical structure of the patient is acquired in a preliminary step that is not specifically included in the method according to the invention.

In this respect, said 3D medical image may be acquired at any time before carrying out this method, by any suitable technique such as Computed Tomography (CT), or Magnetic resonance Imaging (MRI).

In the description that follows, the invention is mainly described with reference to the planning of the implantation of a knee prosthesis, the intervention comprising the implantation of a femoral implant and/or a tibial implant on a patient's knee.

However, the invention is not limited to this kind of implantation and can be implemented for the planning of any other surgical intervention comprising the implantation of an implant.

The anatomical landmarks of the patient if applicable are acquired in a preliminary step that is not specifically included in the method according to the invention.

In this respect, said anatomical landmarks may be acquired at any time before carrying out this method, by any suitable technique such as selecting them in 2D slices of the 3D medical image, or selecting them in reconstructed images wherein each pixel of said reconstructed images integrates the information of a 3D image along a determined direction of integration, said determined direction of integration depending on the axis of the 3D image and possibly on the previously acquired anatomical landmarks.

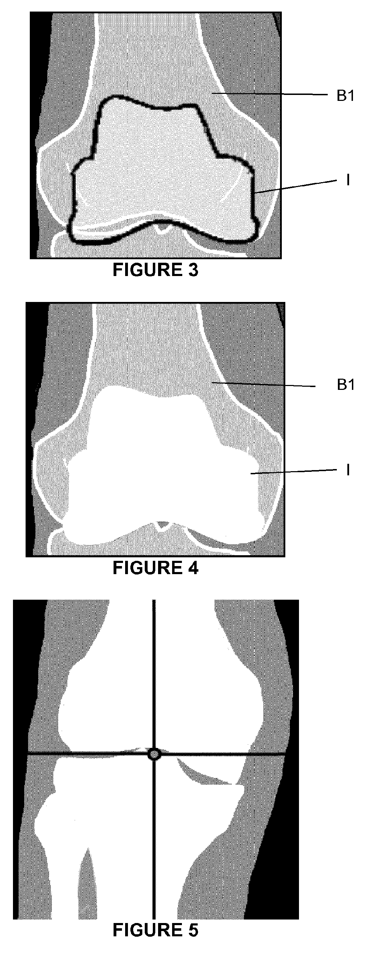

For example, Figure 5 shows a reconstructed image of a knee on which an anatomical landmark such as knee center can be selected (represented by the central circle), wherein the direction of integration can be the Y axis of the 3D image but could also be the vector orthogonal to both the epicondylar and to the Z axis of the 3D medical image.

Said anatomical landmarks can ideally be acquired without sending the 3D medical image to an expert center, for example in the method according to the invention.

An initial planning of the position and orientation of the implant in the referential of the 3D medical image is acquired in a preliminary step that is not specifically included in the method according to the invention.

In this respect, said initial planning may be acquired at any time before carrying out this method, by any suitable technique such as using some default values to position the implant with respect to said anatomical landmarks.

Said initial planning can ideally be acquired without sending the 3D medical image to an expert center, for example in the method according to the invention.

The method can be carried out by a planning system comprising at least one processor that is able to compute and update the pseudo-radiographic images, and a display device, such as a screen, for displaying the pseudo-radiographic images with a representation of the implant.

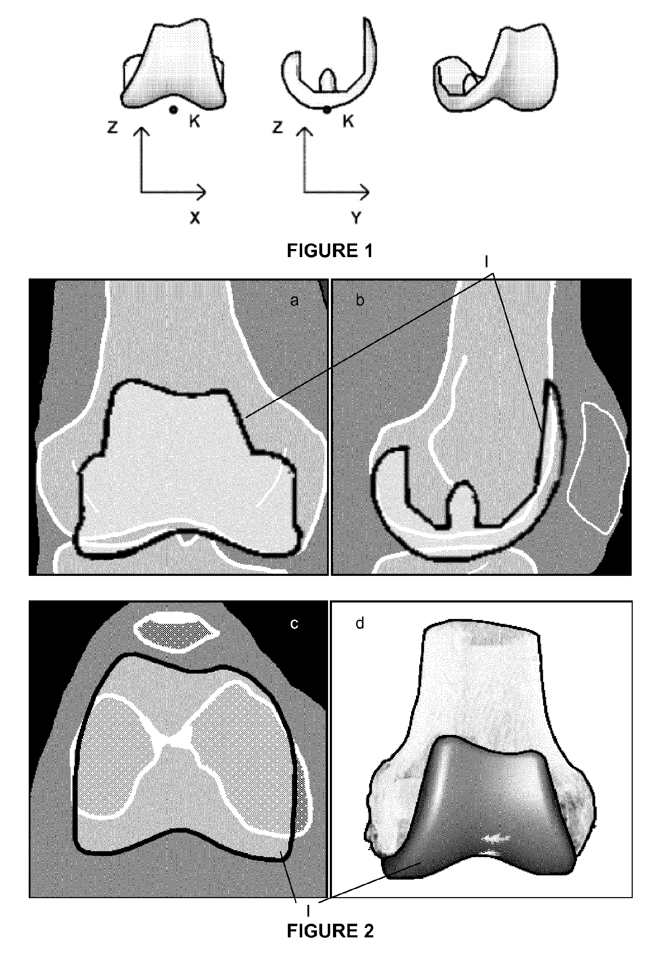

For example, Figure 2 is a schematic view of display that can be obtained with the invention, comprising two pseudo-radiographic images with implant and anatomical structures (a, b), a slice with implant (c), a 3D volume rendered image with some pixels highlighted (d).

The way of computing these images is explained below.

### Determination of a direction of integration

In the method according to the invention, a direction of integration is defined for each of the at least one pseudo-radiographic images, said determination of the direction of integration depending on the planned position of the implant.

In the method according to the invention, said determined direction of integration can be one of the implant's axes.

Figure 1 is a schematic view of an implant (here, a femoral component of a knee prosthesis) in a referential X, Y, Z of the implant, according to three different directions of view.

The axis which could preferably be used in the process of defining a direction of integration is one of axes X, Y and Z.

Some reference points of the implant (e.g. the center K of the knee prosthesis can also be displayed.

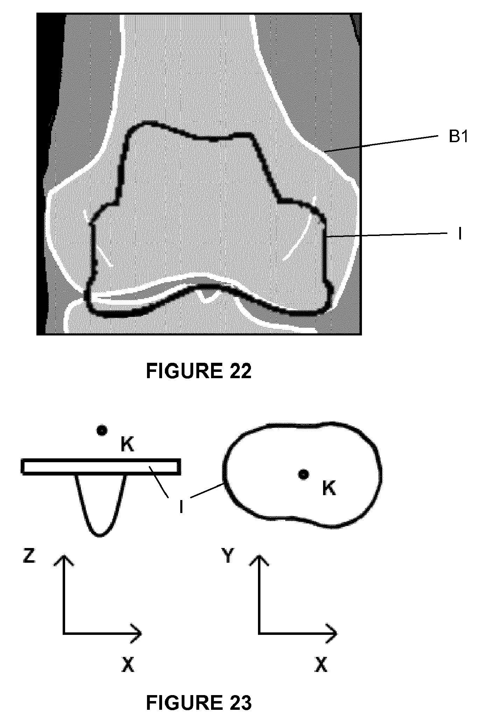

Figure 23 is a schematic view of a tibial component of a knee prosthesis according two different directions of view, showing the axes of this implant and the center K of the knee prosthesis.

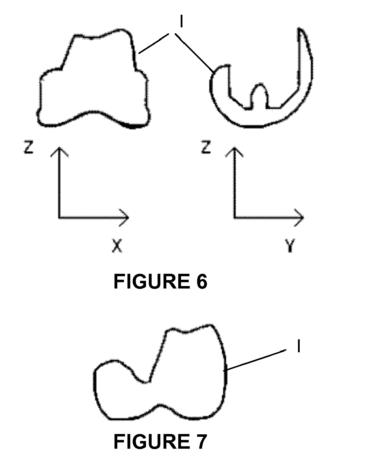

An advantage of having said determined direction of integration be one of the implant's axis is that the resulting image can be better understood by the surgeon than an image with a direction of integration parallel to an axis of the 3D medical image. Indeed, by choosing a direction of integration which is an axis of the implant, frontal and sagittal representations of the implant integrated along the direction of integration which is an axis of the implant would appear familiar to the surgeon (see Figures 6 and 3). On the contrary, if the patient's anatomy is rotated in the 3D medical image, frontal and sagittal representations of the implant integrated along the direction of integration which is an axis of the 3D medical image would not be familiar to the surgeon (see Figure 7).

The direction of integration can be determined by a more complicated formula depending on at least one of the implant's axes or at least one of the implant's reference points, and on zero or more said anatomical landmarks.

Advantages of determining said direction of integration by a more complicated formula depending on at least one of the implant's axes or at least one of the implant's reference points, and on zero or more said anatomical landmarks include the fact that the effect of the modification of the prosthesis position can be better understood by the surgeon. For example, on a sagittal pseudo-radiograph of the knee, it can be interesting to define the Y axis of the image as the direction from prosthesis's K to an anatomical reference such as the hip center H: to define another vector of the image X' as the Y axis of the prosthesis then the X axis of the image as the vector orthogonal to Y image in the plane defined by X', Y image and prosthesis's K; finally to define the direction of integration is then defined as the cross product of X image and Y image. By doing so, the flessum is the angle between X image and the prosthesis cutting plane as seen on the representation of the prosthesis on the image, and when modifying flessum, the pseudo-radiograph will stay still while the implant is turning in the image. Also, the pseudo-radiograph will stay still while changing varus or valgus, while the implant representation slightly changes, and this is what is expected to be seen in real post-surgical radiographs.

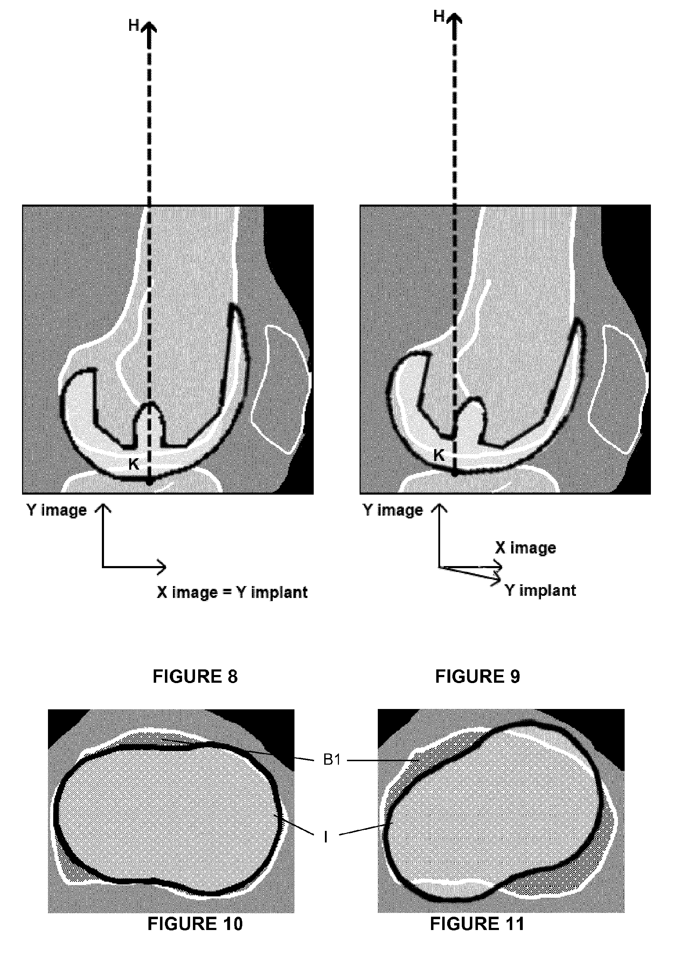

Figure 8 is a zoom of Figure 2 on a sagittal pseudo-radiograph. The Y axis of the image (noted Y_{image}) is the direction from prosthesis's center K to an anatomical reference such as the hip center H. Another vector of the image is the Y axis of the prosthesis (noted Yᵢₘₚₗₐₙₜ). The X axis of the image (noted X_{image}) is the vector orthogonal to Y_{image} in the plane defined by Yᵢₘₚₗₐₙₜ, Y_{image} and prosthesis's center K. The direction of integration is defined as the cross product of X_{image} and Y_{image}, which depends on the position of some anatomical landmarks as well as on the position of the implant. In this case, X_{image} and Yᵢₘₚₗₐₙₜ coincide.

Figure 9 is a sagittal pseudo-radiograph with the same definitions of image axis as in Figure 8, with the same input parameters, including flessum, wherein some varus has been added. In this case, X_{image} and Yᵢₘₚₗₐₙₜ no longer coincide.

### Determination of a 3D image

The method is based on a 3D image of the patient including the anatomical structure onto which an implant is to be implanted.

According to one embodiment, said 3D image can be a 3D medical image directly obtained by Computed Tomography.

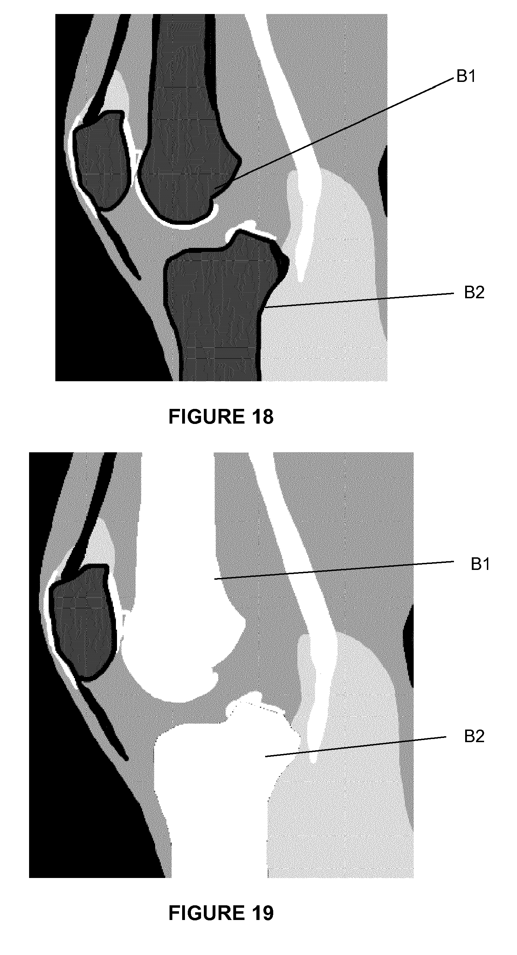

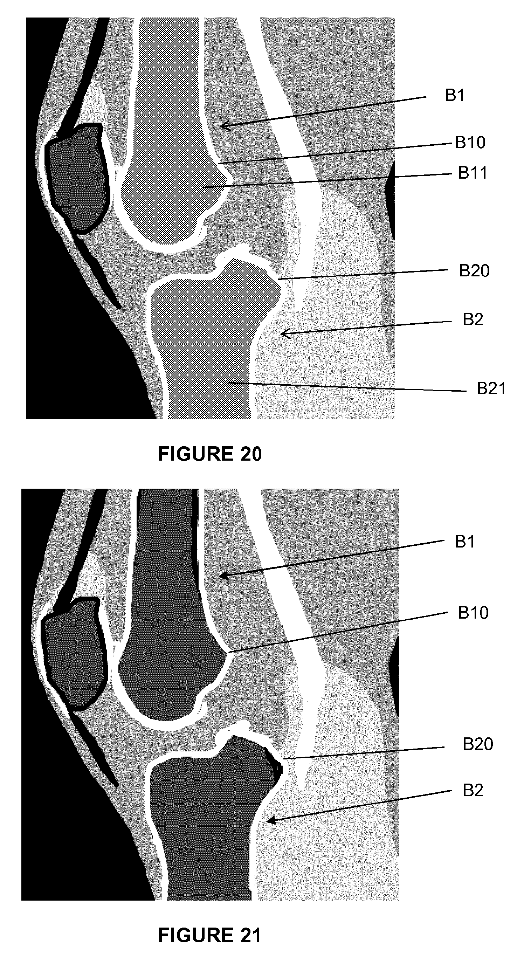

According to an alternative embodiment, said 3D image can be computed as a 3D augmented medical image obtained by applying to a 3D medical image at least one of the following transformations:
- modifying the grey level values of the 3D medical image using a look-up table. A possible advantage of this transformation is that the final image can be made more realistic. Another possible advantage of this transformation can be to prepare the 3D image for other transformations. Another possible advantage of this transformation is that images with a different modality from CT, such as MR images, can be made to look like CT by giving realistic values for a CT exam.
- creating a 3D model of the anatomical structure that may be a bone, or a bone and cartilage, by an automatic segmentation of the 3D medical image, and assigning a grey level value to each voxel of said 3D model. An advantage of this transformation is that images with a different modality from CT, such as MR images, can be made to look like CT. Indeed, in some modalities, the bone is either black or white, the air is black, and the soft tissues are in different shades of grey. Although the accurate segmentation of the cartilage and/or the bone on MR images, usually required for the construction of accurate patient-specific guides, can be tedious and require manual adjustments, a rough segmentation can isolate the bone from the surrounding soft tissues and be sufficient for a realistic pseudo-radiographic image. Such automated segmentation can further be eased with the prior knowledge of the position of anatomical landmarks. The pseudo-radiographic image will look like a projection radiograph. For example, Figure 18 is a schematic view showing a native MR image of a joint comprising two bones B1, B2, whereas Figure 19 is the image of figure 18 wherein both bones B1, B2 have been segmented and all pixels of bone have been replaced by a value (here white).
- creating a 3D model of the anatomical structure by an automatic segmentation of the 3D medical image, and assigning a grey level value to each voxel of said 3D model using a priori models of the anatomical structure, said a priori models comprising cortical bone models and spongious bone models. For example by giving a value close to the Hounsfield unit of cortical bone to the pixels in the periphery of the segmentation, and a value close to the Hounsfield unit of spongious bone elsewhere. For example, Figure 20 is the image of Figure 18 wherein both bones B1, B2 have been segmented and the respective lining B10, B20 has been replaced by a value (the same white as cortical bone) and the inners B11, B21 have been replaced by a different value (the same as spongious bone). An advantage of this transformation is that images with a different modality from CT, such as MR images, can be made to look like CT. Indeed, in some modalities, the bone is either black or white, the air is black, and the soft tissues are in different shades of grey. Although the accurate segmentation of the cartilage and/or the bone, usually required for the construction of accurate patient-specific guides, can be tedious and require manual adjustments, a rough segmentation can isolate the bone from the surrounding soft tissues and be sufficient for a realistic pseudo-radiographic image. Such automated segmentation can further be eased with the prior knowledge of the position of anatomical landmarks. The pseudo-radiographic image will look like a projection radiograph even more than assigning the same grey level value to each voxel of said 3D model.
- creating a 3D model of the anatomical structure by an automatic segmentation of the 3D medical image, and assigning grey level values to the external surface of said 3D model. For example, Figure 21 is the image of figure 18 wherein the bones B1, B2 have been segmented and the respective lining B10, B20 has been replaced by a value (white), whereas the inners have not been replaced. An advantage of this transformation is that images with a different modality from CT, such as MR images, can be made to look like CT. Indeed, in some modalities, the bone is either black or white, the air is black, and the soft tissues are in different shades of grey. Although the accurate segmentation of the cartilage and/or the bone, usually required for the construction of accurate patient-specific guides, can be tedious and require manual adjustments, a rough segmentation can isolate the bone from the surrounding soft tissues and be sufficient for a realistic pseudo-radiographic image. Such automated segmentation can further be eased with the prior knowledge of the position of anatomical landmarks. The pseudo-radiographic image will look like a projection radiograph even more than assigning the same grey level value to each voxel of said 3D model.

The acquisition of the 3D medical image from which the 3D image is determined is carried out prior to the planning method according to the invention and thus does not form part of the invention itself. Any technique for acquiring a 3D medical image may be used. After its acquisition, the 3D medical image may be stored in a memory or another physical support such as a CD-ROM.

### Computation of a pseudo-radiographic image

One or more pseudo-radiographic images are computed by integrating the information of the 3D image along said determined direction of integration, for example by using one or more of the following transformations:
- Summing the value of pixels along said determined direction of integration.
- Maximum intensity projection
- Using more complex formulae for more realistic projections that simulate the physics of X-ray transmission.
- Use of other mathematical functions, such as look up tables, before or after application of other transformations

Said integration may take into account the whole 3D image, or only part of the information such as a five cm-strip around the implant.

### Display of a pseudo-radiographic image with a representation of the implant

Said one or more pseudo-radiographic images are displayed with a representation of the implant (see Figures 2a and 2b).

Such representation of the implant must display the implant where it is planned in the image, but there are pros and cons to the different ways of displaying the implant. Examples of ways to display the implant include:
- Projection radiograph of the implant: the implant is displayed in opaque white as it would appear in post-surgical radiographs. An advantage is that the final image will look like post-surgical radiographs the surgeon can be familiar with. Figure 4 shows a coronal pseudo-radiograph of the knee wherein the implant I is displayed in opaque white on a bone B1 (here, the femur), as it would appear on post-surgical radiographies.
- Transparent projection radiograph of the implant: the implant is displayed in transparent color (white or other color) as it would appear in post-surgical radiographs. An advantage is that the final image will look almost like post-surgical radiographs the surgeon can be familiar with, and yet he can see the anatomy behind the prosthesis. Figure 3 is a zoom of figure 2 on a coronal pseudo-radiograph of the knee wherein the implant I is displayed in transparent color on the bone B1.
- Lining of the implant: the implant inners are displayed transparent (fully transparent, or in transparent color) and its lining is displayed in opaque color. An advantage is that the surgeon can see the anatomy behind the prosthesis, but the final image look less like post-surgical radiographs the surgeon can be familiar with. Figure 22 shows a coronal pseudo-radiograph of the knee wherein the lining of the implant I is displayed on the bone B1.

The choice of the display could be a choice which could be modified interactively in the method according to the invention or prior to the method according to the invention.

### Display of a slice of the 3D image with a representation of the implant

In addition to the display of the pseudo-radiograph(s), at least one slice of the 3D image may be displayed with a representation of the implant (see Figure 2c). Like the pseudo-radiographs, the slice position and orientation can be defined using:
- at least one of the implant's axis or at least one of the implant's reference points,
- a more complicated formula depending on at least one of the implant's axis or at least one of the implant's reference points, and on zero or more said anatomical landmarks. An advantage of this definition is that the controls are more natural to the surgeon. The effect of the modification of the prosthesis position can be better understood by the surgeon. For example, on an axial slice of the tibia, it can be interesting to define the Z axis of the image (its normal) as the Z axis of the implant; to define the Y axis of the image as the projection of patient's tibia referential; to define the X axis of the image as the cross product of Y image with Z image. By doing so, the slice stays still when the surgeon modifies the external rotation while the implant representation rotates on the display unit. Figure 10 is an axial slice with the representation of a knee implant I and a bone B2, whereas Figure 11 is an axial slice with the representation of the knee implant with the same parameters for the position of the implant as in Figure 10, wherein some external rotation has been added.

The 3D image used to compute the slice is not necessarily the same as the 3D image used to compute the pseudo-radiographic image. Indeed, it can be an advantage to keep the 3D native medical image so that the surgeon better understands the slice.

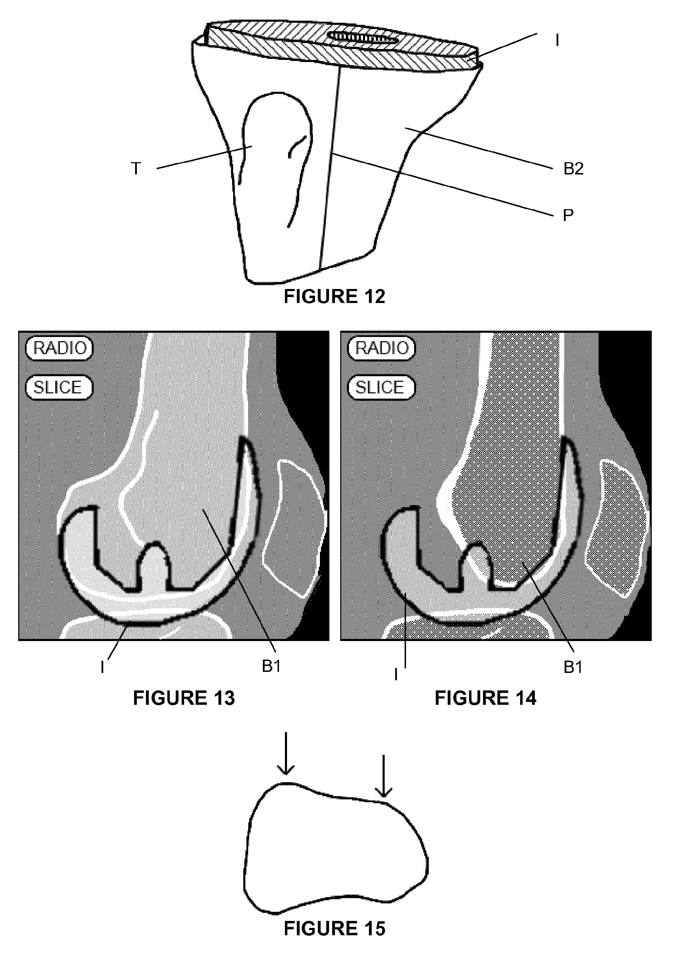

In a preferred embodiment of the method, a way is provided to alternatively display a slice of the 3D image (see Figure 13) or a pseudo-radiographic image (see Figure 14) showing a bone B1 and a representation of an implant I, wherein the slice and the pseudo-radiographic image share the same orientation. There are a number of known ways for doing so, such as buttons, mouse clicks, mouse hovering, or use of other devices such as pedals linked to the computer unit.

An advantage of providing said way to alternatively display a slice of the 3D image or a pseudo-radiographic image is that screen space is saved and that the representation of the implant can be the same in both views. In practice, for a knee implant, it is important that the femur component anterior cutting plane exits the cortical bone, ideally at the top of the prosthesis. This is hard to see this on a radio as the anterior cortical bone is not flat (there are two bumps, as shown by the arrows on Figure 15) and is better seen on a slice. On the other hand, the position of the prosthesis with respect to the anatomical condyles cannot be seen on that slice while it is understood on the pseudo-radiographic image.

### Display of a volume rendering image with a representation of the implant

In a preferred embodiment, at least one volume rendering of the anatomical structure 3D image is displayed with a representation of the implant (see Figure 2d). An advantage of displaying said at least one volume rendering of the anatomical structure with a representation of the implant is that the surgeon can have the global view that he would have preoperatively. It thus reduces the risk of gross malposition of the implant. Figure 12 illustrates an example of a volume rendering of bone B2 displayed with a representation of the implant I.

### Display of reference feature

In a preferred embodiment, one or more reference features can be displayed on at least one volume rendering of the 3D image. For example, as illustrated in Figure 12, which shows a tibial implant I on a 3D volume rendering of the tibia B2, the plane P defined by prosthesis K, Y implant and Z implant (see Figure 13) can be highlighted by displaying the plane in opaque or transparent color, or the pixels of that plane can be set to a color which stands out. An advantage of doing so is that the position of the implant with respect to anatomical landmarks visible on the volume rendering can be seen. An example of this is to see the position of the anterior tibia tuberosity T with respect to the plane P defined by prosthesis K, Y implant and Z implant.

### Display of anatomical landmarks

In a preferred embodiment, one or more anatomical landmarks are displayed in the images. For example, a point can be displayed in the position it would have in the pseudo-radiographic image. An advantage of doing so is that displayed information such as the resection level of a knee implant can be better understood.

### Display of controls

In a preferred embodiment of the method according to the invention, one or more controls are displayed to modify interactively the position of the implant. Some controls can be displayed or used directly on the at least one pseudo-radiographic images, or on a slice of the 3D image, or on a volume rendering of the 3D image.

There are a number of ways of displaying controls on the interface, such as buttons, or clicking and dragging on the implant to translate it, or click and dragging around the implant to rotate it.

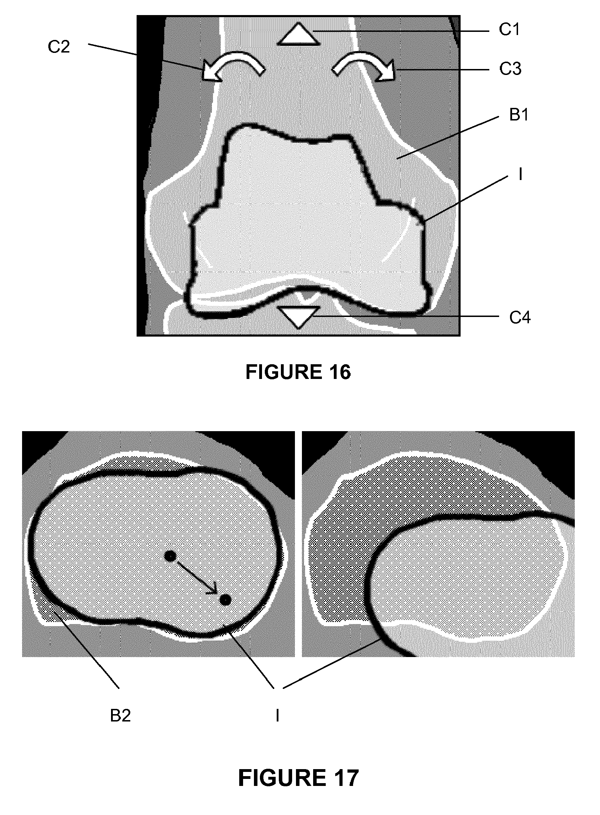

For example, Figure 16 is a pseudo-radiographic image with the representation of an implant I on a bone B1, wherein controls C1, C2, C3 and C4 are displayed on the pseudo-radiographic image.

Figure 17 shows a slice of the 3D image with the representation of an implant I on a bone B2, wherein clicking and dragging on the implant (see left figure, the mouse move being represented by the arrow between the 2 black points) translates the implant accordingly (see right figure).

An advantage is that screen space is saved because there is no need to have some space for controls. Another advantage is that the surgeon sees all the information that matters in the same place, so he can focus on this place. Another advantage is that written information such as figures, controls, and visual information (prosthesis on the patient's anatomy) are grouped together, which also makes for a better understood interface.

### Update of the display

In the method according to the invention, when the position of the implant is modified, the display is updated accordingly, which comprises:
- the computation of one or more updated directions of integration that depend on the modified position of the implant;
- the computation of one or more updated pseudo-radiographic images each along a said updated direction of integration
- the display of said one or more updated pseudo-radiographic images with an updated representation of the implant.

Other elements displayed can be updated too, such as 2D slices of a 3D image with the representation of the implant, volume rendering image with a representation of the implant, reference features, anatomical landmarks, controls and information if applicable.

It is possible that some elements need not be modified when the position of the implant is modified. For example, for the planning of a knee surgery, the sagittal pseudo-radiographic image for tibia planning does not need to be modified when the slope is modified. The representation of the implant in the sagittal pseudo-radiographic image for tibia planning does not need to be modified when the implant is moved laterally or medially.

## Claims

1. Method for planning a surgical intervention comprising the implantation of an implant in a patient's anatomical structure, comprising:
- computing at least one pseudo-radiographic image from a 3D image of the anatomical structure, said pseudo-radiographic image being a 2D image wherein each pixel integrates the information of the 3D image along a determined direction of integration, said determined direction of integration depending on the planned position of the implant with respect to the anatomical structure;
- displaying said at least one pseudo-radiographic image on a display unit;
- displaying a representation of the implant on said pseudo-radiographic image;
- updating the pseudo-radiographic image and/or the representation of the implant when the position of the implant is modified.

2. Method according to claim 1, wherein the 3D image is a 3D medical image directly obtained by Computed Tomography.

3. Method according to claim 1, wherein the 3D image is a 3D augmented medical image obtained by applying to a 3D medical image at least one of the following transformations:
- modifying the grey level values of the 3D medical image using a look-up table,
- creating a 3D model of the anatomical structure by an automatic segmentation of the 3D medical image, and assigning a grey level value to each voxel of said 3D model,
- creating a 3D model of the anatomical structure by an automatic segmentation of the 3D medical image, and assigning a grey level value to each voxel of said 3D model using a priori models of the anatomical structure, said a priori models comprising cortical bone models and spongious bone models,
- creating a 3D model of the anatomical structure by an automatic segmentation of the 3D medical image, and assigning grey level values to the external surface of said 3D model.

4. Method according to claim 3, wherein the 3D medical image is a magnetic resonance image.

5. Method according to one of claims 1 to 4, wherein said determined direction of integration is a specific direction of the implant, such as one of the three axes of the implant referential.

6. Method according to one of claims 1 to 4, wherein said determined direction of integration is defined by a specific direction of the implant and by at least one anatomical parameter such as a mechanical axis of a bone on which the implant shall be implanted.

7. Method according to one of claims 1 to 6, comprising computing at least two pseudo-radiographic images according to different directions of integration and displaying on the same display unit said at least two pseudo-radiographic images and a representation of the implant on each of said images.

8. Method according to one of claims 1 to 7, further comprising computing at least one slice of a 3D image and displaying a representation of the implant on said slice.

9. Method according to claim 8, wherein said slice is computed according to the same direction as the determined direction of integration of the pseudo-radiographic image and wherein the method further comprises using a window for alternatively displaying the pseudo-radio image and said slice of the 3D image on the display unit.

10. Method according to one of claims 1 to 9, further comprising computing volume rendering of the 3D image and displaying said computed image with a representation of the implant on the same display unit as the at least one pseudo-radiographic image.

11. Method according to claim 10, further comprising highlighting a reference feature of the implant on the volume rendering computed image.

12. Method according to one of claims 1 to 11, further comprising displaying selected anatomical landmarks on the pseudo-radiographic image.

13. Method according to one of claims 1 to 12, further providing control elements for interactively modifying the position of the implant.

14. Method according to claim 13, wherein said control elements are displayed on the at least one pseudo-radiographic image.

15. Method according to one of claims 1 to 14, wherein the implant is a femoral or a tibial component of a knee prosthesis.
